# EUROPÄISCHE PATENTSCHRIFT

(11) **EP 2 415 449 B1**
(45) Veröffentlichungstag und Bekanntmachung des Hinweises auf die Patenterteilung: **23.08.2017**
(21) Anmeldenummer: 11167818.1
(22) Anmeldetag: 27.05.2011
(51) Int. Cl.: A61K 8/37, A61Q 15/00

(54) **Deodorant- oder Antitranspirantzubereitung mit Polyolpartialester**
Deodorant or anti-transpirant preparation with polyol partial esters
Préparation de déodorant ou d'antitranspirant dotée d'ester partiel de polyol

(30) Priorität: 31.05.2010 DE 102010022064
(43) Veröffentlichungstag der Anmeldung: 08.02.2012
(73) Patentinhaber: Beiersdorf AG, 20253 Hamburg (DE)
(72) Erfinder: Miertsch, Heike, 22459 Hamburg (DE); Biel, Stefan, 21077 Hamburg (DE); Timmerbeil, Myriam, 30539 Hannover (DE)

(56) Entgegenhaltungen:
- EP-A2- 0 319 126
- EP-A2- 1 250 917
- EP-A2- 2 363 387
- WO-A1-2005/097044
- DE-A1- 2 413 697
- DE-A1-102005 029 388
- DE-A1-102008 013 023
- US-A1- 2009 208 437

## Beschreibung

Die Erfindung beschreibt kosmetische oder dermatologische Deodorant- oder Antitranspirantzubereitungen mit Polyolpartialestern, gebildet aus mindestens einem Polyol mit 3 bis 6 Kohlenstoffatomen enthaltend 2 bis 6 OH-Gruppen und mindestens einer Carbonsäure enthaltend 5 bis 18 Kohlenstoffatome.
Die Polyolpartialester weisen ein bestimmtes Verhältnis von ursprünglichen im Polyol vorhandenen freien Hydroxygruppen zu veresterten Hydroxygruppen auf.

Üblicherweise werden Antitranspirantien (AT) und Deodorantien (Deo) in mannigfaltigen Produktformen angeboten, wobei in Europa Roller, Pumpzerstäuber und Aerosole dominieren, in den USA, Mittel- und Südamerika eher die Stifte. Es sind sowohl wasserfreie (Suspensionen) als auch wasserhaltige Produkte (hydro-alkoholische Formulierungen, Emulsionen) bekannt.

Im Stand der Technik finden sich bei wasserfreien Suspensionen fast ausschließlich Deodorant- oder Antitranspirantfomulierungen, die Silikonöle, insbesondere Cyclomethicone, beinhalten.
Silikonöle, insbesondere Cyclomethicone, dienen als Trägeröl für die Wirkstoffe und ermöglichen so ein gleichmäßiges Aufbringen auf die Haut. Durch die hohe Flüchtigkeit dampft Cyclomethicon sehr schnell von der Haut ab ohne ein klebriges Gefühl zu verursachen. Durch das Abdampfen des Silikonöls werden weiße Rückstände durch das AT-Mittel oder die Verdicker auf der Haut sehr schnell sichtbar (Weißeleffekt). Formeln mit Cyclomethiconen werden durch viele Verbraucher als zu trocken und unangenehm im Hautgefühl empfunden, da sie kein Pflegegefühl vermitteln.

Weiterer Nachteil silikonhaltiger Deo- bzw. AT-Aerosole ist, dass das Verdicken von Silikonölen, insbesondere Cyclomethicon, nur mit ausgewählten Rohstoffen möglich ist. Teilweise werden dadurch höhere Mengen an Verdickern benötigt, die somit den Weißeleffekt erhöhen.
Ein Nachteil Cyclomethicon-haltiger AT- bzw. Deo-Zubereitungen ist auch, dass durch das schnelle Abdampfen der flüchtigen Komponenten auch Parfüminhaltsstoffe entfernt werden, was zu einem Duftverlust bzw. einem nicht ausreichenden "long-lasting"-Effekt führt und die Parfümierung erschwert.

Lipide bezeichnen Fette und fettähnliche Stoffe. Für die Kosmetik sind sie vor allem als weichmachende ("emollient") Inhaltsstoffe und als hauteigene Lipide der Hornschicht, die zwischen den Hornzellen lagern, von Bedeutung. Sie befähigen die Haut zur Speicherung von Feuchtigkeit. Neben dem pflegenden Aspekt werden Lipide den kosmetischen Zubereitungen zugesetzt um eine bessere Verteilbarkeit auf der Haut zu gewährleisten und um die sensorischen Eigenschaften der Zubereitungen zu verbessern. In vielen kosmetischen Zubereitungen sind aus diesen Gründen leichtflüchtige Silikonöle zugesetzt. Insbesondere Cyclomethicone werden aus diesen Gründen den kosmetischen Zubereitungen zugefügt.
Cyclomethicone, z.B. INCI-Bezeichnung für ein Octamethylcyclotetrasiloxan, besitzen die allgemeine Formel, mit n=3-6

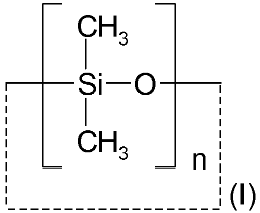

Angeboten werden beispielsweise Cyclomethicon/Decacyclopentasiloxan (D5) oder Cyclomethicon/Dodecamethylcyclohexasiloxan (D6). Die Gemische können für sich allein oder in entsprechenden Zubereitungen als flüchtige Silicon-Verbindungen eingesetzt werden (Cosmet. Toiletries 107, Nr. 5, 27 [1992]).

Nachteile der Cyclopentasiloxane (D5) und der Silikonöle allgemein sind
a) Verfügbarkeit
   Durch hohen Verbrauch von Silikonölen in der Photovoltaik und in der Baustoffindustrie ist eine permanente Versorgung mit Silikonölen für die Kosmetik nicht ungehindert gewährleistet.
b) Nachhaltigkeit
   Silikonöle zeichnen sich häufig durch eine mangelnde Abbaubarkeit aus.
c) Weißeleffekt
   Silikonöl-haltige Formulierungen zeigen den Nachteil des Weißelns von Antitranspirantien auf insbesondere schwarzer Baumwoll-Kleidung. Silikonöl-freie Formulierungen mit gleicher Konzentration des Antitranspirant-Wirkstoffs in Form eines Pulvers zeigen ein deutlich geringeres Weißeln.
d) Pflegegefühl
   Silikonöle in Kosmetika vermitteln dem Verbraucher eher ein negatives Pflegegefühl. Silikonöle haben auch deutliche Schwächen in Bezug auf Ihre Kompatibilität mit kosmetischen Wirkstoffen. Aufgrund ihres stark hydrophoben Charakters vermögen Silikonöle insbesondere Wirkstoffe mit polarem Charakter kaum zu lösen. Weiterhin sind Silikonöle praktisch nicht in der Lage, die Penetration kosmetischer Wirkstoffe in die Haut zu unterstützen.

In der vorliegenden Erfindung bestand daher die Aufgabe Deo- oder Antitranspirantzubereitungen zur Verfügung zu stellen, die die Nachteile der silikonölhaltigen Formulierungen vermindern bzw. vermeiden. Insbesondere ist es wünschenswert Cyclomethicon-freie Formulierungen zu entwickeln, die dennoch sehr gute sensorische Eigenschaften haben, keine flüchtigen Öle enthalten, die Packmittel-kompatibel (insbesondere Packmittel aus PP) sind und vor allem keine Einbußen hinsichtlich der Deo-/AT-Wirksamkeit zeigen.

Neben den kosmetischen Zubereitungen gibt es eine Vielzahl an medizinischdermatologisch topischen Zusammensetzungen, die in der Regel ein oder mehrere Arzneistoffe in wirksamer Konzentration enthalten. Auch diese Zusammensetzungen bedürfen der Verbesserung hinsichtlich ihrer Verteilbarkeit auf der Haut, ihrer sensorischen Eigenschaften als auch Deo-/AT-wirksamkeit. Zur besseren Unterscheidung zwischen kosmetischer und medizinischer Anwendung und entsprechenden Produkten werden auf die gesetzlichen Bestimmungen der Bundesrepublik Deutschland verwiesen (z.B. Kosmetikverordnung, Lebensmittel- und Arzneimittelgesetz).

Die DE 19631004 und DE 19543696 beschreiben kosmetische Zubereitungen umfassend ein oder mehrere Substanzen, gewählt aus der Gruppe der Monoglycerin-monocarbonsäuremonoester, der Diglycerin-monocarbonsäuremonoester, der Triglycerin-monocarbonsäuremonoester, der Monoglycerin-dicarbonsäure-monoester, der Diglycerin-dicarbonsäure-monoester und der Triglycerin-dicarbonsäure-monoester.

Darin genannte Ester sind Glycerinmonocaprylat (GMCy, Glycerinmonocaprinat GMC, Glycerinmonostearat GMS, Glycerinmonoundecylat GMU, Diglycerinmonocaprinat DMC, Triglycerinmonolaurat TML, Triglycerinmonomyristat TMM.
Der Anteil an diesen Estern beträgt 0,1 bis 10 Gew.% in den Formulierungen der DE 19631004 bzw. DE 19543696.

Ein Verfahren zur Herstellung von Carbonsäureestern wird in der DE 3818293 beschrieben. Darin werden auch Difettsäurediglycerinester mit definierter Hydrophilie offenbart, die gegenüber Diglycerinmonoestern oder höheren Polyglycerinestern eine verbesserte Stabilität von W/O-Emulsionen erzeugen.

EP 1762216 offenbart flüssige make-up Entferner mit nichtionischen Tensiden und Glycerinpartialestern einer C6 bis C12 Fettsäure, wobei der Gesamtanteil an Mono- und Diestern mehr als 50 % beträgt und das Gewichtsverhältnis der Mono- zu Diestern 4 oder weniger beträgt.

EP 1800650 beschreibt Hydroxyester aus der Veresterung eines Polyols mit C4-C16 Säuren, mit einem hohen Anteil an Monoestern.

EP 531684 beschreibt Kombinationen von Glycerindiestern mit insgesamt 13 bis 20 C-Atomen mit Polyalkoholen und Tensiden für Reinigungsmittel. Die Tensidmenge in dem Reinigungsmittel beträgt dabei 10 bis 50% und ist mindestens doppelt so hoch gewählt wie die Menge des Glycerindiesters.

EP 277335 beschreibt sogenannte "Milky Lotion" in denen die Verwendung von Glycerindicaprylaten in Kombination mit nichtionischem Tensid, wie beispielsweise PEG-10 Oleat, in kosmetischen Formulierungen offenbart werden. Die Offenbarung ist den EP 1800650 und EP 1762216 gleich.

EP 522624 offenbart Glyceryl Caprylate und Glyceryl Caprate als bevorzugte Emulgatoren für spezielle Lippenstift-W/O-Emulsion.

US 6265372 beschreibt Kombinationen von Glycerindiestern mit C3-C12 Fettsäuren mit Siliconen und Tensiden für Reinigungsmittel, wobei die Tensidmenge mindestens so hoch ist wie die Menge des Glycerindiesters.

DE 102008013023 beschreibt ein Verfahren zur Herstellung von Octansäureglycerinestern mit einem Molverhältnis Glycerin zu Octansäure im Bereich 1 zu 1 bis 1 zu 0,45.

Nachteil bekannter Partialester ist deren feste bis pastöse Eigenschaft. Besonders Monoglyceride mit einem geringen Anteil an Diestern sind bei Raumtemperatur fest oder pastös, während Triglyceride auf der anderen Seite flüssig sind.

Es ist bekannt, dass Glycerinpartialester mit einem hohen Monoglycerid-Anteil gut geeignet sind, die Penetration kosmetischer Wirkstoffe in die Haut zu unterstützen. Typische Produkte, für die eine solche Wirkung ausgelobt wird, sind beispielsweise AKOLINE

Produkte der Firma Karlshamns. Die AKOLINE Produkte sind Partialester des Glycerins mit Capryl/Caprinsäure mit einem Monoglyceridanteil > 50%. Ein ähnlich ausgelobtes Produkt ist beispielsweise IMWITOR 308 der Firma Sasol. Dabei handelt es sich um einen Partialester des Glycerins mit Caprylsäure mit einem Monoglyceridanteil > 80 %.

Sowohl das Produkt IMWITOR 308 (Schmelzpunkt 30-34 °C) als auch die AKOLINE-Produkte (Schmelzpunkt 25 - 28 °C) sind bei Raumtemperatur feste bzw. pastöse kristalline Massen. Eine Verwendung solcher festen Glycerinpartialester mit einem Monoglyceridanteil > 50% als kosmetische Öle mit guter Verteilbarkeit und leichtem Hautgefühl ist daher unmöglich.

Weitere Beispiele für bei RT feste Monoglyceride sind Glycerylmonostearat und Glycolmonostearate. Beispiele für pastöse Monoglyceride sind Akoline MCM von Aarhus Karlsham (INCI: Caprylic/ Capric Glycerides), IMWITOR 742 (INCI: Caprylic/ Capric Glycerides), IMWITOR 928 (INCI: Glyceryl Cocoate) oder IMWITOR 988 (INCI: Caprylic Glycerides).

Weiterer Nachteil der Partialglyceride ist, dass deren Einsatzmenge in kosmetischen Produkten begrenzt ist, da, in hoher Konzentration eingesetzt, sie einen Einfluss auf die Viskosität haben. Eine hohe Einsatzmenge ist daher bei festen Partialglyceriden wie Glycerylmnonostearate nicht möglich. Der Einsatz einer hohen Konzentration an Glycerylmonostearat würde die Viskosität einer Formulierung deutlich erhöhen. Selbst bei festen Produktformen wie den wasserfreien Antitranspirans-Stiften würde eine hohe Konzentration von Glycerylmonostearat die Festigkeit des Stifts deutlich erhöhen, den Abrieb des Produkts auf der Haut reduzieren und somit eine vergleichbare Produktapplikation nicht mehr ermöglichen.

Wünschenswert sind daher Partialglyceride, die man auch in einer hohen Konzentration einer Formulierung zufügen kann, ohne dass sich die Viskosität der Wirkstofflösung der Aerosole oder die Konsistenz wasserfreier Antitranspirans-Stifte ändert.

Es wurde überraschend gefunden, dass kosmetische oder dermatologische Deodorant- oder Antitranspirant-Zubereitungen umfassend ein oder mehrere Polyolpartialester gebildet aus einem Polyol mit 3 bis 6 Kohlenstoffatomen enthaltend 2 bis 6 OH-Gruppen und mindestens zwei verschiedenen Carbonsäuren enthaltend 5 bis 10 Kohlenstoffatome, wobei das molare Verhältnis der OH-Gruppen im Polyol und den Acylgruppen der Carbonsäuren im Polyolpartialester zwischen 1 zu 0,90 und 1 zu 0,35 liegt, eine vorteilhafte Lösung der geschilderten Probleme darstellt.

Die erfindungsgemäßen Polyolpartialester sind Veresterungsprodukte eines Polyols, ausgewählt aus Polyolen mit 3 bis 6 Kohlenstoffatomen enthaltend 2 bis 6 OH-Gruppen, bevorzugt 3 bis 6 OH-Gruppen, mit mindestens zwei verschiedenen Carbonsäuren enthaltend 5 bis 10 Kohlenstoffatome, bevorzugt 6 bis 10, besonders bevorzugt 8 bis 10 Kohlenstoffatome, mit der Maßgabe, dass das molare Verhältnis der OH-Gruppen im Polyol und den Acylgruppen der Carbonsäuren im Polyolpartialester zwischen 1 : 0,90 und 1 : 0,35, bevorzugt zwischen 1 : 0,83 und 1 : 0,40 und besonders bevorzugt zwischen 1 : 0,70 und 1:0,45 liegt. Polyolpartialester mit einem Veresterungsgrad im Bereich um 50% ist besonders bevorzugt.

Ein Polyolpartialester mit einem Veresterungsgrad von 50% bedeutet, dass 50% freie Hydroxidgruppen im Ester vorliegen, die anderen 50% der OH-Gruppen sind durch die Carbonsäure oder -säuren verestert.

D.h. der Veresterungsgrad der erfindungsgemäßen Polyolpartialester liegt im Bereich von 35 bis 90 Mol.%, bevorzugt 40 bis 83 Mol.%, insbesondere im Bereich 45 bis 70 Mol% und ganz besonders vorteilhaft im Bereich um 50 Mol.%, bezogen auf die Gesamtmolzahl an veresterbaren Hydroxidgruppen des Polyols im Ester.

Entscheidend ist das Verhältnis der tatsächlich sich in der Mischung der Polyolpartialester befindlichen OH-Gruppe zu den Acylgruppen, den veresterten Hydroxidgruppen. Unter dem Begriff der "Polyolpartialester" werden im Zusammenhang mit der vorliegenden Erfindung bevorzugt Mischungen mindestens zweier verschiedener Polyolester verstanden, die sich durch ihren Veresterungsgrad unterscheiden.

So ist beispielsweise ein reiner Mono- oder Triglycerinester kein erfindungsgemäßer Polyolpartialester. Reiner Monoglycerinester enthält 66 Mol.% an unveresterten, freien Hydroxidgruppen, und damit 34% an Acylgruppen, reiner Triester enthält 0 Mol.% an unveresterten Hydroxidgruppen, und damit 100% an Acylgruppen, so dass deren Veresterungsgrad jeweils ausserhalb des erfindungsgemäßen Bereiches von 35 - 90% liegt. Als Hydroxygruppe (OH-Gruppe) im Polyol ist die Anzahl der OH-Gruppen im eingesetzten und umgesetzten Polyol gemeint. D.h. die Anzahl nicht veresterter Hydroxidgruppen.

Die Anzahl Acylgruppen bezieht sich auf die veresterten Säuregruppen im Partialester. D.h. erfindungsgemäße Polyolpartialester weisen einen Molanteil an veresterten Hydroxidgruppen (Acylgruppen) von 35 bis 90 Mol.% auf, in Bezug zu den insgesamt vorhandenen veresterbaren Hydroxidgruppen.

Das bedeutet umgekehrt, dass 10 bis 65 Mol.% freie, unveresterte Hydroxidgruppen im erfindungsgemäßen Partialester enthalten sind.

So weist beispielsweise das bevorzugte erfindungsgemäße Partialglycerid der Capryl- und Caprinsäure, als Caprylic/Capric Glyceride bezeichnet, ein Anteil an freien OH-Gruppen im Bereich von 50% auf.

Erreicht wird das bestimmte erfindungsgemäße Verhältnis der OH-Gruppen zu Acylgruppen durch die Synthesebedingungen der Polyolpartialester, bei denen die Säure im Unterschuss zugegeben wird.

Folgende Synthesebeispiele verdeutlichen die Herstellung erfindungsgemäßer Polypartialester, wobei erfindungsgemäß sind nur die Polypartialester, die aus mindestens einem Polyol mit 3 bis 6 Kohlenstoffatomen enthaltend 2 bis 6 OH-Gruppen und mindestens zwei verschiedenen Carbonsäuren enthaltend 5 bis 10 Kohlenstoffatome dadurch gekennzeichnet, dass der Veresterungsgrad der Polyolpartialester im Bereich von 35 Mol% bis 90 Mol% liegt, bezogen auf die Gesamtmolzahl an veresterbaren OH-Gruppen.

### Versuch A:

In einen 2l Vierhalskolben werden 139,9 g (1,5207 mol) Glycerin und 360,1 g (2,2852 mol) Vorlauffettsäure eingewogen und unter Verwendung von 0,5 g Zinnoxalat sowie 0,25 g H3PO3 bei gleichzeitigem N2-Strom bis auf 240 °C erhitzt. Unter diesen Bedingungen lässt man nun solange rühren, bis die SZ <3 ist. Danach wird der Ansatz abgekühlt, filtriert und abgefüllt.

| | |
|---|---|
| Brechungsindex 20°C: | 1,4518 |
| Farbzahl Hazen: | 33 |
| SZ: | 0,5 mg KOH/g |
| GC-Verteilung (Flächen-%): freies Glycerin | 5,3 % |
| Monoglyceride | 34,8 % |
| Diglyceride | 46,4 % |
| Triglyceride | 13,5 % |

D.h. Versuch A zeigt die Herstellung erfindungsgemäßer Polyolpartialester, eine Polyolpartialestermischung, mit 40,8 Mol.% an freien unveresterten OH-Gruppen und 59,2 Mol.% an Acylgruppen. D.h. einem Verhältnis von ursprünglichen OH-Gruppen zu Acylgruppen von 1 : 0,592. Nicht umgesetztes Glycerin und dessen unveresterte Hydroxidgruppen werden nicht berücksichtigt.

### Versuch B:

In einen 2l Vierhalskolben werden 422,4 g (4,5913 mol) Glycerin und 1077,6 (6,8865 mol) Vorlauffettsäure eingewogen und unter Verwendung von 0,99 g Zinnoxid und 0,75 g H3PO3 bei gleichzeitigem N2-Strom bis auf 240 °C erhitzt. Unter diesen Bedingungen lässt man nun solange rühren, bis die SZ <1 ist. Danach wird der Ansatz abgekühlt, filtriert und abgefüllt. Kennzahlen:

| | |
|---|---|
| OHZ: | 242 mgKOH/g, |
| VZ: | 284 mgKOH/g, |
| SZ: | 0,1 mgKOH/g |
| Brechungsindex 20°C: | 1,4524 |
| Farbzahl Hazen: | 30 |

### Versuch C:

In einen 11 Vierhalskolben werden 141,8 g (1,5413 mol) Glycerin und 358,2 g (3,0836 mol) 2-Ethylbuttersäue eingewogen und unter gleichzeitigem N2-Strom bis auf 240 °C erhitzt. Unter diesen Bedingungen lässt man nun solange rühren, bis die SZ <1 ist. Danach wird der Ansatz abgekühlt und abgefüllt.

| | |
|---|---|
| Brechungsindex 20°C: | 1,5242 |
| Farbzahl Hazen: | 130 |
| SZ: | 0,3 mgKOH/g |

### Versuch D:

In einen 1l Vierhalskolben werden 172,8 g (1,8783 mol) Glycerin und 327,2 g (2,8168 mol) 2-Ethylbuttersäue eingewogen und unter gleichzeitigem N2-Strom bis auf 240 °C erhitzt. Unter diesen Bedingungen lässt man nun solange rühren, bis die SZ <1 ist. Danach wird der Ansatz abgekühlt und abgefüllt.

| | |
|---|---|
| Brechungsindex 20°C: | 1,4468 |
| Farbzahl Hazen: | 186 |
| SZ: | 1,0 mgKOH/g |

### Versuch E:

In einen 4l Vierhalskolben werden 568,2 g (6,176 mol) Glycerin und 2431,8 g (15,432 mol) Vorlauffettsäure eingewogen und unter Verwendung von 3 g Zinnoxalat sowie 1,5 g H3PO3 bei gleichzeitigem N2-Strom bis auf 240 °C erhitzt. Unter diesen Bedingungen lässt man nun solange rühren, bis die SZ <1 ist. Danach wird der Ansatz abgekühlt, filtriert und abgefüllt.

**Kennzahlen:**

| | |
|---|---|
| OHZ: | 50 mgKOH/g, |
| VZ: | 324 mgKOH/g, |
| SZ: | 0,8 mgKOH/g |
| Brechungsindex 20°C: | 1,4493 |
| Farbzahl Hazen: | 30 |
| GC-Verteilung (Flächen-%): freies Glycerin | 0,0 % |
| Monoglyceride | 2,4 % |
| Diglyceride | 30,1 % |
| Triglyceride | 67,5 % |

D.h. Versuch E zeigt die Herstellung erfindungsgemäßer Polyolpartialester, eine Polyolpartialestermischung, mit 11,6 Mol.% an freien unveresterten OH-Gruppen und 88,6 Mol.% an Acylgruppen. D.h. einem Verhältnis von ursprünglichen OH-Gruppen zu Acylgruppen von 1 : 0,886, einem Veresterungsgrad von 88,6%.

### Versuch F:

In einen 2l Vierhalskolben werden 339 g (3,6848 mol) Glycerin und 1161 g (7,3677 mol) Vorlauffettsäure eingewogen und unter Verwendung von 1,5 g Zinnoxalat sowie 0,75 g H3PO3 bei gleichzeitigem N2-Strom bis auf 240 °C erhitzt. Unter diesen Bedingungen lässt man nun solange rühren, bis die SZ <3 ist. Danach wird der Ansatz abgekühlt und abgefüllt.

**Kennzahlen:**

| | |
|---|---|
| OHZ: | 134 mgKOH/g, |
| VZ: | 315 mgKOH/g, |
| SZ: | 2,5 mgKOH/g |
| Brechungsindex 20°C: | 1,4504 |
| Farbzahl Hazen: | 169 |

Der Vorteil der erfindungsgemäßen Ester wird durch die bestimmte Anzahl an freien Hydroxidgruppen erreicht, die im Bereich von 10 - 65 Mol.%, bevorzugt 17 - 60 Mol%, insbesondere im Bereich 30 - 55 Mol%, bezogen auf die Gesamtmolzahl an ursprünglichen Hydroxidgruppen im eingesetztem und umgesetzten Polyol, liegt.

Durch die freien Hydroxidgruppen wird die Polarität der Polyolpartialester gegenüber vollveresterten Estern erhöht. Das hat den Vorteil, dass die erfindungsgemäßen Partialester in kosmetischen oder dermatologischen Formulierungen quasi als Emulgator fungieren können und somit die Stabilität von klassischen O/W- und W/O-Emulsionen erhöhen. Unter dem Begriff "Polyolpartialester" werden im Zusammenhang mit der vorliegenden Erfindung daher bevorzugt Mischungen mindestens zweier verschiedener Polyolester verstanden, die sich durch ihren Veresterungsgrad unterscheiden.

So kann ein beispielsweise erfindungsgemäßer Glycerinpartialester mindestens zwei der Glycerinester enthalten, ausgewählt aus Glycerinmonoester, Glycerindiester und Glycerintriester, sofern das erfindungsgemäße Molverhältnis an Hydroxidgruppen zu Acylgruppen gegeben ist.

Der Vollester ist in diesem Zusammenhang als möglicher Bestandteil der Mischung explizit miterfasst. Der Vollester alleine ist jedoch nicht erfindungsgemäß. Besonders bevorzugt sind die zwei Carbonsäuren zu wählen aus 2-Ethylbutansäure, 2-Ethylhexansäure, n-Octansäure und n-Decansäure, insbesondere n-Octansäure und n-Decansäure.

Weiter bevorzugt werden erfindungsgemäße Polyolpartialester als Mischung eines Polyols mit mindestens zwei verschiedenen Carbonsäuren gebildet.

Bevorzugte Polyole sind Zucker, wie Pentosen und Hexosen, und Zuckeralkohole, sowie deren Anhydride, insbesondere solche ausgewählt aus der Gruppe umfassend, bevorzugt bestehend aus Sorbitol, Mannitol, Xylitol, Erythritol, Arabitol, Sorbitan und einfache Polyole wie insbesondere Pentraerythrit, Trimethylolmethan, Trimethylolethan, Trimethylolpropan, 1,2,4-Butantriol, 1,2-Propandiol, 1,3-Propandiol und Glycerin, wobei Glycerin in diesem Zusammenhang erfindungsgemäß besonders bevorzugt ist.

Zum Erhalt der Polyolpartialester werden insbesondere unsubstituierte, lineare oder verzweigte Carbonsäuren eingesetzt. Diese sind erfindungsgemäß bevorzugt gesättigt, jedoch kann es auch für bestimmte Fragestellungen vorteilhaft sein, aromatische Carbonsäuren einzusetzen, insbesondere Benzoesäure.

Bevorzugt eingesetzte Carbonsäuren zum Erhalt der Polyolpartialester sind insbesondere gesättigte, keine Heteroatome enthaltende Carbonsäuren. Insbesondere umfasst die bevorzugte Gruppe Neopentansäure, Isoamylsäure, Pentansäure, n-Hexansäure, 2-Ethylbutansäure, Cyclohexancarbonsäure, n-Oktansäure, 2-Ethylhexansäure, iso-Oktansäure, Isononansäure, 3,5,5-Trimethylhexansäure, n-Decansäure, iso-Decansäure, Laurinsäure und 2-Butyloctansäure, besonders bevorzugt 2-Ethylbutansäure, 2-Ethylhexansäure, n-Octansäure und n-Decansäure, insbesondere n-Octansäure und n-Decansäure.
Es ist erfindungsgemäß besonders bevorzugt, dass pro Mol im Polyol ursprünglich enthaltener OH-Gruppe insgesamt 0,45 bis 0,70 mol der OH-Gruppen mit Acylgruppen der Carbonsäure verestert im Polyolpartialester vorliegen.
Insbesondere sind solche Polyolpartialestermischungen bevorzugt, bei denen jeder einzelne, in der Mischung enthaltene Polyolester eines bestimmten Veresterungsgrades (wie beispielsweise -monoester, -diester, -triester) nicht mehr als 80 Gew.-%, bevorzugt nicht mehr als 70 Gew.-% und besonders bevorzugt nicht mehr als 60 Gew.-%, bezogen auf die Gesamtmasse der Polyolpartialestermischung, ausmacht.

Die erfindungsgemäßen Verhältnisse der unveresterten Hydroxidgruppen zu den veresterten Gruppen stellt sich bei der Synthese automatisch durch das Molverhältnis des Polyols zu der Säure ein, wie die Herstellbeispiele A bis F zeigen.
Es werden dabei nicht verschiedene Partialglyceride miteinander gemischt, sondern das Verhältnis stellt sich durch die Synthese-Bedingungen ein.

Es ist dem Fachmann offenbar, dass zur Herstellung der Polyolpartialester Mischungen von Polyolen und/oder Mischungen der Carbonsäuren eingesetzt werden können.
In diesem Zusammenhang ist eine besonders bevorzugte Ausführungsform dadurch gekennzeichnet, dass zum Erhalt des Polyolpartialesters eine Mischung aus n-Octansäure und n-Decansäure in einem Gewichtsverhältnis von 40 bis 80 Gew.% (n-Octansäure) zu 20 bis 60 Gew.% (n-Decansäure), bevorzugt von 50 bis 70 Gew.% zu 30 bis 50 Gew.%, insbesondere von 55 bis 65 Gew.% zu 35 bis 45 Gew.%, eingesetzt werden, bezogen auf die Gesamtmasse an Octansäure und Decansäure.

Dabei ist es besonders bevorzugt, dass pro Mol im Polyol enthaltener OH-Gruppe insgesamt 0,45 bis 0,70 mol, bevorzugt 0,48 bis 0,52 mol Acylgruppen der Octansäure und Decansäure verestert im Polyolpartialester vorliegen. In diesem Zusammenhang ist das eingesetzte Polyol insbesondere Glycerin.

Die erfindungsgemäßen Polyolpartialester sind weiter erhältlich nach dem Fachmann an sich bekannten thermischen Verfahren wie beispielsweise beschrieben in der DE102008013023, WO 9811179, EP 407959, Ullmann's Encyclopedia of Industrial Chemistry, 6th ed. 2002, Kapitel "Esters, Organic" oder enzymatischen Verfahren wie beschreiben in US6613551 und U. T. Bornscheuer in "Enzyme and Microbial Technology", 17, 578-586, 1995.

Demnach werden das Polyol und die Carbonsäure unter dem Fachmann bekannten Bedingungen für Veresterungsreaktionen, gegebenenfalls in Anwesenheit eines Katalysators, durchgeführt. Insbesondere wird die Veresterung unter Entfernung von Wasser aus dem Reaktionsgemisch durchgeführt. Das thermische Verfahren zum Erhalt der Polyolpartialester wird bevorzugt bei 120-260 °C, besonders bevorzugt 160-250°C durchgeführt. Im Fall einer enzymatisch katalysierten Veresterungsreaktion ist die Verfahrenstemperatur entsprechend auf einen Bereich von 20 bis 80 °C, bevorzugt 30 - 60 °C anzupassen. Der Reaktionsverlauf kann beispielsweise über die Säurezahl, beispielsweise nach dem Verfahren der DIN53402 oder der DIN EN ISO 2114, des Produktes überwacht werden, so dass es bevorzugt ist, die Reaktion so lange durchzuführen, bis die gewünschte Säurezahl erreicht wird.

Die so erhaltenen erfindungsgemäßen Polyolpartialester sind dadurch gekennzeichnet, dass sie bei 1 bar Druck einen Schmelzpunkt von kleiner 22 °C, insbesondere kleiner 20 °C aufweisen. D.h. die erfindungsgemäßen Partialester sind bei Temperaturen oberhalb 22°C flüssig und nicht, wie die aus dem Stand der Technik bekannten Ester, fest oder pastös.

Es ergibt sich daraus ein weiterer Vorteil der erfindungsgemäßen Partialester, indem die Mischbarkeit mit anderen Ölphasen gegeben ist ohne ein Schmelzen oder Erwärmen durchführen zu müssen.

Damit ist auch eine einfache (Kalt-)Herstellung der kosmetischen Zubereitungen möglich. Dies wiederum führt zu einer schonenderen Herstellbarkeit, was bei temperaturempfindlichen Inhaltsstoffen vorteilhaft ist.

Bevorzugte Partialester sind erhältlich aus:
2. 1 mol Glycerin mit 1 mol Caprylsäure und 1 mol Benzoesäure
3. 1 mol Glycerin mit 1 mol Caprylsäure und 1,5 mol Benzoesäure
4. 1 mol Glycerin mit 2 mol C8/10 Fettsäure
5. 1 mol Glycerin mit 1,5 mol C8/10 Fettsäure und 1 mol Benzoesäure
6. 1 mol Glycerin mit 1 mol Benzoesäure und 1 mol C8/10 Fettsäure
7. 1 mol Glycerin mit 1 mol Benzoesäure und 1 mol Isononansäure
8. 1 mol Glycerin mit 1 mol Benzoesäure und 1 mol 2 Propylheptansäure
9. 1 mol Glycerin mit 0,8mol Benzoesäure und 0,8 mol C8/10 Fettsäure
10. 1 mol Glycerin mit 1 mol Benzoesäure und 1 mol 2 Propylheptansäure
12. 1 mol Glycerin mit 0,5 mol Benzoesäure und 1,5 mol C8/10 Fettsäure
13. 1 mol Glycerin mit 1,5 mol Benzoesäure und 0,5 mol C8/10 Fettsäure
14. 1 mol Glycerin mit 0,25 mol Benzoesäure und 1,75 mol C8/10 Fettsäure
15. 1 mol Glycerin mit 0,50 mol Benzoesäure und 1,50 mol Isononansäure
17. 1 mol Trimethylolpropan mit 1 mol Benzoesäure und 1 mol C8/10 Fettsäure
19. 1 mol Trimethylolpropan mit 2 mol C8/10 Fettsäure
22. 1 mol Glycerin mit 0,5 mol Isononansäure und 1,5 mol Benzoesäure
24. 1 mol Glycerin mit 1 mol 2-Ethylbuttersäure und 1 mol Benzoesäure
27. 1 mol Glycerin mit 2,5 mol Fettsäure C8/10
28. 1 mol Glycerin mit 1,5 mol Fettsäure C8/10
37. 1 mol 1,2Pentandiol mit 1 mol C8/10 Fettsäure
39. 1 mol 1,2-Octandiol mit 1 mol C8/10 Fettsäure

Besonders bevorzugte Partialester sind erhältlich aus:
4) 1 mol Glycerin mit 2 mol C8/10 Fettsäure
27) 1 mol Glycerin mit 2,5 mol C8/10 Fettsäure
28) 1 mol Glycerin mit 1,5 mol C8/10 Fettsäure
wie auch in folgender Tabelle dargestellt

| Polyol | n O H | Acid A | DE A | %OH Esterified | RI (20°C) | visosity [mPas] (25°C) | surface tenson [mN/m ] | interfacial tension [mN/m] | density 25°C |
|---|---|---|---|---|---|---|---|---|---|
| Glyceri n | 3 | C8/10 | 2,5 0 | 83% | 1,449 6 | 26,3 | 28,8 | 7,3 | 0,949 |
| Glyceri n | 3 | C8/10 | 2,0 0 | 67% | 1,450 2 | 57,0 | 28,4 | 2,7 | 0,962 |
| Glyceri n | 3 | C8/10 | 1,5 0 | 50% | 1,451 8 | 56,5 | 27,9 | n.a. | 0,978 |

Die ebenfalls unter diesem Namen Caprylic/Capric Glycerides erhältlichen Marktprodukte weisen jedoch zum Unterschied zu den erfindungsgemäßen Estern einen deutlich höheren Anteil an Monoester auf, bzw. die Summe Mono- und Diester ist deutlich höher als bei dem erfindungsgemäßen Caprylic/Capric Glyceride. D.h. auch, dass die freie OH-Zahl der erfindungsgemäßen Ester deutlich niedriger ist gegenüber den aus dem Stand der Technik bekannten Partialestern.

Die erfindungsgemäßen Polyolpartialester können in Antitranspirant-Produkten in sehr hoher Konzentration eingesetzt werden ohne dass sie einen Einfluss auf die Viskosität haben. Das ist bei festen Partialglyceriden wie Glycerylmnonostearate nicht möglich. Ein Einsatz von einer hohen Konzentration an Glycerylmonostearat würde die Viskosität einer Formulierung deutlich erhöhen. Selbst bei festen Produktformen wie den wasserfreien Antitranspirans-Stiften würde eine hohe Konzentration von Glycerylmonostearat die Festigkeit des Stifts deutlich erhöhen, den Abrieb des Produkts auf der Haut reduzieren und somit eine vergleichbare Produktapplikation nicht mehr ermöglichen. Diese Nachteile werden mit Einsatz der erfindungsgemäßen Polyolpartialester vermieden.

Ein weiterer Unterschied zu den Partialestern des Standes der Technik ist neben dem erfindungsgemäßen Verhältnis an unveresterten OH-Gruppen zu veresterten OH-Gruppen zusätzlich auch über das besonders bevorzugte Verhältnis von Mono-/Di- und Trieester in der Partialestermischung gegeben. Bevorzugt werden Verhältnisse von Mono- und Diestern zu Triestern im Bereich von 9 : 1 bis 1 : 9, besonders bevorzugt sind Verhältnisse im Bereich von 4 : 1 bis 1 : 8, gewählt.
Vorteilhaft können die erfindungsgemäßen Partialester zu einem Gewichtsanteil im Bereich von 3 bis zu 90 Gew.%, bezogen auf die Gesamtmasse der Zubereitung, gewählt werden.

Bevorzugt ist der Anteil der Polyolpartialester in AT-Stiften im Bereich bis zu 60 Gew%, insbesondere bis zu 10 Gew%, bezogen auf die Gesamtmasse der Zubereitung. Bei den Aerosol-Sprays beträgt der bevorzugte Gewichtsanteil der erfindungsgemäßen Partialester 10 bis 60 Gew.%, bezogen auf die Gesamtmasse der Wirkstofflösung, wobei die Einsatzmenge bezogen auf die Gesamtmasse der Zubereitung von dem Treibgasanteil abhängt.

Diese hohen Einsatzmengen an Polyolpartialestern sind in den Stand der Technik Deo-/AT-Zubereitungen aufgrund der dargelegten Gründe nicht vorzufinden.
Die Marktprodukte sind in der Regel eher fest/wachsartig/pastös und nicht bei Raumtemperatur flüssig wie die erfindungsgemäßen Polyolpartialester.

Weitere Vorteile der erfindungsgemäßen Partialester ergeben sich beim Einsatz in kosmetischen oder dermatologischen Deodorant- oder Antitranspirantzubereitungen.

Antitranspirantien oder Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

Im allgemeinen Sprachgebrauch erfolgt nicht immer ein klare Trennung der Begriffe "Deodorant" und "Antitranspirant". Vielmehr werden - insbesondere auch im deutschsprachigen Raum - Produkte zur Anwendung im Achselbereich pauschal als Desodorantien bzw. "Deos" bezeichnet. Dies geschieht unbeachtlich der Frage, ob auch eine antitranspirante Wirkung vorliegt.

Antitranspirantien (AT) sind schweißverhütende Mittel, die - im Gegensatz zu den Desodorantien, die im Allgemeinen eine mikrobielle Zersetzung von bereits gebildetem Schweiß verhindern - die Absonderung von Schweiß überhaupt verhindern sollen.

Die gewünschte Minimierung der Schweißsekretion kann durch verschiedene Mechanismen realisiert werden. Hierzu zählt traditionell der Einsatz von Adstringenzien, welche Eiweißfällungen und Gerinnungen hervorrufen und so eine Verengung oder Verschluss der Schweißdrüse bewirken.

Neuartige AT-Wirker basieren bspw. auf dem Prinzip der Anticholinergika, welche die Nervenreize, die zur Sekretionsteigerung der Schweißdrüsen führen, unterbrechen.
Ein weiteres Prinzip neuartiger AT-Wirker beruht auf der Beeinflussung von Membrantransportvorgängen in der Zelle. So hemmen spezifische Aquaporin-Inhibitoren die Proteine die Kanäle in der Zellmembran bilden, um den Durchtritt von Wasser und weiteren Molekülen zu erleichtern. Auch lonenkanal-Hemmer bewirken eine Beeinflussung von Membrantransport- bzw. Osmose-Vorgängen. Ein weiterer neuartiger AT-Wirkmechanismus lässt sich durch die Verwendung kurzkettiger, vicinaler Diole realisieren, welcher möglicherweise auf deren osmotischen Aktivität zurückzuführen ist.

So durch Anwendung eines Mittels kein Einfluss auf die Schweißsekretion ausgeübt, also keine antitranspirante Wirkung realisiert wird, liegt erfindungsgemäß kein Antitranspirant vor. Erfindungsgemäß sind demnach als Antitranspirantwirkstoffe solche Stoffe umfasst, die einen Einfluss auf die Schweißsekretion haben.

Im Gegensatz zu den Antitranspirantien bewirken reine Desodorantien keine aktive Beeinflussung der Schweißsekretion, sondern lediglich die Steuerung bzw. Beeinflussung des Körper- bzw. Achselgeruches (Geruchsverbesserungsmittel). Gängige Wirkmechanismen hierzu sind antibakterielle Effekte, wie sie auch das nicht-kolloidale Silber zeigen, Geruchsneutralisation (Maskierung), Beeinflussung von bakteriellen Metabolismen, die reine Parfümierung wie auch die Verwendung von Vorstufen bestimmter Parfümkomponenten, welche durch enzymatische Umsetzung zu wohlriechenden Stoffen umgesetzt werden.

Zubereitungen können neben den eigentlichen schweißhemmenden Wirkstoffen (AT-Wirker) zusätzlich auch Stoffe enthalten, die den mikrobiellen Abbau des Schweißes hemmen, wie z.B. Triclosan. Triclosan wirkt gegen Gram-positive und Gram-negative Keime sowie gegen Pilze und Hefen, woraus eine desodorierende, jedoch keine antitranspirante Wirkung resultiert, da aus der Beeinflussung der bakteriellen Hautflora keine Beeinflussung der Schweißsekretion abzuleiten ist.

Insbesondere bei Antitranspirantien auf Basis von Aluminiumsalzen kann ggf. auf den weiteren Zusatz von rein desodorierend wirkenden Substanzen verzichtet werden, da diese per se ein antimikrobielles Potential aufweisen. Des Weiteren wird durch das reduzierte Wasserangebot durch die verminderte Schweißsekretion auch das bakterielle Wachstum gehemmt.

Antitranspirantwirkstoffe im Sinne der vorliegenden Anmeldung sind insbesondere aus den folgenden Gruppen zu wählen.

Als Antitranspirantwirkstoffe finden insbesondere Adstringenzien Verwendung, vornehmlich Aluminium-Verbindungen. Die früher eingesetzten, stark sauer wirkenden Salze Aluminiumsulfat oder -chlorid und die Agaricinsäure sind weitgehend durch Aluminiumhydroxychlorid und -alkoholate ersetzt worden. Die nachfolgende Auflistung vorteilhaft einzusetzender Antitranspirant-Wirker soll in keinster Weise einschränkend sein: Aluminium-Salze:
- Aluminium-Salze wie Aluminiumchlorid AlCl3, Aluminiumsulfat Al2(SO4)3
- Aluminiumchloride der empirischen Summenformel [Al2(OH)mCln], wobei m+n=6
- Aluminiumchlorhydrat [Al2(OH)5Cl] x H2O
   ▪ Standard Al-Komplexe: Locron P (Clariant), Micro-Dry (Reheis), ACH-331 (Summit), Aloxicoll PF 40 (Giulini).
   ∘ Aktivierte Al-Komplexe: Reach 501 (Reheis), AACH-324 (Summit), AACH-7171 (Summit), Aloxicoll P (Giulini), Aloxicoll SD100
- Aluminiumsesquichlorhydrat [Al2(OH)4,5Cl1,5] x H2O
   ∘ Standard Al-Komplexe: Aluminum Sesquichlorohydrate (Reheis), AACH-308 (Summit)
   ∘ Aktivierte Al-Komplexe: Reach 301 (Reheis)
- Aluminiumdichlorhydrat [Al2(OH)4Cl2] x H2O

### Aluminium-Zirkonium-Salze:

• Aluminium/Zirkonium Trichlorhydrex Glycin [Al4Zr(OH)13Cl3] x H2O x Gly
   ∘ Standard Al/Zr-Komplexe: Rezal 33GP (Reheis), AZG-7164 (Summit), Zirkonal P3G (Giulini)
   ∘ Aktivierte Al/Zr-Komplexe: Reach AZZ 902 (Reheis), AAZG-7160 (Summit), Zirkonal AP3G (Giulini)
• Aluminium/Zirkonium Tetrachlorhydrex Glycin [Al4Zr(OH)12Cl4] x H2O x Gly
   ∘ Standard Al/Zr-Komplexe: Rezal 36G (Reheis), AZG-368 (Summit), Zirkonal L435G (Giulini)
   ∘ Aktivierte Al/Zr-Komplexe: Reach 908 (Reheis), AAZG-7167 (Summit), Zirkonal AP4G (Giulini)
• Aluminium/Zirkonium Pentachlorhydrex Glycin [Al8Zr(OH)23Cl5] x H2O x Gly
• Aluminium/Zirkonium Octachlorhydrex Glycin [Al8Zr(OH)20Cl8] x H2O x Gly

Ebenso von Vorteil können aber auch Glycin-freie Aluminium/Zirkonium-Salze sein.

Vorteilhaft kann auch die Verwendung von AT-Salz-Suspensionen bzw. -Gelen sein, bei denen pulverförmig vorliegende Aluminium-Salze in diversen Ölen dispergiert angeboten werden.

Ebenso kann ein antimikrobieller Silbercitratkomplex, wie er in der DE 202008014407 beschrieben ist, bevorzugt als kosmetisch wirksamer Bestandteil in den kosmetischen Zubereitungen eingesetzt werden.

Die Antitranspirant-Wirkstoffe aus den zuvor geschilderten Gruppen werden in den erfindungsgemäßen Formulierungen bevorzugt in einer Menge von 1 bis 30 Gew.%, bezogen auf die Gesamtmasse der Zubereitung, d.h. inklusive der ggf. vorhandenen Treibgase, eingesetzt.
Bei Einsatz von ca. 35 Gew.% AACH (aktiviertes Aluminium Chlorohydrat) in der Wirkstofflösung für ein Aerosolspray (ohne Treibgas) und einem Abfüllverhältnis von etwa 15 : 85 (Wirkstofflösung zu Treibgas) wird ein Anteil von etwa 5,25 Gew. % AACH im Endprodukt vorhanden sein.

AT-mittel aus der Gruppen der Anticholinergika, wie beispielsweise 4-[(2-Cyclopentyl-2-hydroxyphenylacetyl)oxy]-1,1-dimethyl-piperidiniumsalze, insbesondere das 4-[(2-Cyclopentyl-2-hydroxyphenylacetyl)oxy]-1,1-dimethyl-piperidiniumbromid können zu einem Anteil von bevorzugt 0,05 bis 1,0 Gew.%, vorzugsweise 0,1%-0,7%, insbesondere 0,3%-0,5 Gew.%, bezogen auf die Gesamtmasse der Zubereitung zugesetzt werden.
Auch vicinale Diole und ähnliche Wirkstoffe aus der Gruppe der osmotisch aktiven Substanzen können als AT-wirker den erfindungsgemäßen Zubereitungen zugesetzt werden. Selbstverständlich ist es möglich weitere oder andere Antitranspirant Wirkstoffe und/oder Deodorantien als kosmetisch wirksamen Bestandteil zu zusetzen.

Bevorzugt wird als kosmetisch wirksamer Bestanteil ein oder mehrere Verbindungen aus der Gruppe der Deodorantien, insbesondere Parfüme, Alkohole, antimikrobielle und/oder antitranspirant wirksame Wirkstoffe gewählt.

Ein Vorteil der neuen Partialester ist, dass die erfindungsgemäßen Partialester Öle sind und bei Raumtemperatur flüssig sind. Sie lassen sich daher z.B. mit Mineralölen mischen oder als Ölkörper in die Zubereitungen einarbeiten. Im Gegensatz zu den Emulgatoren ist der Anteil an Monoestern gering.
Bei der Verwendung in kosmetischen Formulierungen zeichnen sich die erfindungsgemäßen Polyolpartialester insbesondere durch ein nichtöliges, nicht klebriges, trockenes Hautgefühl aus, was sie interessant macht als nichtflüchtige Ersatzstoffe für Cyclomethicone.
Ein weiterer Vorteil der erfindungsgemäßen Polyolpartialester ist, dass sie als Ölkörper in kosmetischen Formulierungen mit Wirkstoffen verwendet werden können und dabei für eine bessere Wirksamkeit der eingesetzten Wirkstoffe sorgen können, da sie eine verbesserte Penetration der Wirkstoffe in die Haut bewirken. Ein großer Vorteil zu bekannten Penetrationsverstärkern wie Glyceryl Monocaprylat (festes Produkt) ist dabei die sehr große Kompatibilität der Polyolpartialester mit typischen unpolaren kosmetischen Ölen wie z.B. Mineralöl.

Die Vorteile und Besonderheiten werden nachfolgend am Beispiel des Partialglycerids der Capryl-/ Caprinsäure mit einem Veresterungsgrad von 1.5 dargestellt.

Bessere antitranspirante Langzeit-Wirkung im Vergleich zu Cyclopentasiloxane im ATP: ATP-Methode bedeutet ein Silikon-Imprint, eine bildanalytische Bestimmung der Schweißproduktion am inneren Unterarm zum Screening oder zur Optimierung von AT-Rohstoffen und Formulierungen hinsichtlich ihrer Wirksamkeit "in vivo".

### Vorgehensweise:

- 5 Tage Konditionierung
- Tag 1 - 2: Auftragung der Produkte
- Produkte werden mit semiokklusiven Pflaster auf markierte Areale der Unterarme aufgetragen und für 4 Std. getragen
- Tag 3:Thermostimulation in der Sauna zur Ermittlung der Anzahl aktiver Schweißdrüsen
- 28 Std. nach letzter Produktapplikation erfolgt ein Saunagang
- Areale werden vor dem Saunagang mit Kleberingen markiert, nach Einsetzen der Schweißsekretion an den Unterarmen werden diese mit Zellstoff abgetupft und Silikon aufgetragen (Aushärtezeit: ∼ 15 Min.).
- Die Imprints werde nach der Abnahme für ca. 24 Stunden zum vollständigen Trocknen bei RT aufbewahrt.
- Danach folgt die bildanalytische Auswertung.

### Probandenkollektiv:

30 Probanden (15 w/15 m) mit physiologisch normalem Schweißfluß, denen der Saunaaufenthalt keine gesundheitlichen Probleme bereitet und deren inneren Unterarme nach 10 - 15 minütigem Aufenthalt in der Sauna (T=75°C; rel. Feuchte ∼25%) eine Schweißsekretion aufweisen.

### Produktanwendung:

10-minütige Akklimatisierung der offenen Unterarminnenseiten vor jeder Applikation unter Standardbedingungen.

2-tägige kontrollierte Applikation der Testprodukte und Kontrollen durch semiokklusive Pflaster auf die Testareale nach kontrolliertem Waschen der inneren Unterarme mit unparfümierter Seife ohne AT-Inhaltsstoffe.

### Areal: innere (volare) Unterarme

### Meßzeitpunkte:

Nach 2 Tagen Produktapplikation; die Probenahme erfolgt 28 Std. nach der letzten Applikation durch Anfertigung eines Silikon-Abdrucks der Testareale; die Silikonmasse wird erst aufgetragen, nachdem der Proband während eines Saunaaufenthaltes eine ausreichende Transpiration an den Unterarmen aufweist; während des Aushärtevorganges hinterlassen die Schweißtröpfchen jeweils Abdrücke im Silikon
Der Test ist geeignet als Screeningtool bzw. zur Optimierung von AT-Rohstoffen und Formulierungen.

Mit dem Test wird ermittelt, ob die Schweißsekretion an den Unterarmen durch die Produkte signifikant zum unbehandelten Areal reduziert wird. Weiterhin können signifikante Unterschiede zwischen den Produkten ermittelt werden.

| AT-Spray [Gew.%] | Vergleichsprodukt | Neuentwicklung |
|---|---|---|
| INCI (Wirkstofflösung/ohne Treibgas) | A | B |
| Octyldodecanol | 1 | 1 |
| Dimethicone | 3 | 3 |
| Disteardimonium Hectorite | 4 | 4 |
| Cyclomethicone (D5) | 50.75 | |
| Caprylic/Capric Glyceride (Veresterungsgrad 50%) | | 50.75 |
| Aluminum Chlorohydrate | 35 | 35 |
| Parfum | 6.25 | 6.25 |
| AT-Wirksamkeit: Mean Restaktivität (Schweißdrüsen) im ATP | 70 % | 55 % |

Zubereitung A stellt die Zubereitung mit Cyclomethicon D5 dar und Zubereitung B die erfindungsgemäße AT-Zubereitung mit erfindungsgemäßen Partialester Caprylic/Capric Glyceride mit einem Veresterungsgrad von 50%.
Es zeigt sich, dass die erfindungsgemäße Zubereitung (B) eine signifikant bessere AT-Wirksamkeit aufweist nur allein durch den Austausch des Cyclomethicons gegen erfindungsgemäße Partialester.

Der Einsatz der erfindungsgemäßen Partialester in kosmetischen oder dermatologischen Deo- oder AT-Zubereitungen führt überraschenderweise damit zu einer Verbesserung der Antitranspirantwirksamkeit.
Die erfindungsgemäßen Polyolpartialester führen entgegen dem Cyclomethicon auch dazu, dass die Formulierung länger auf der Achselhaut verbleibt und der Wirkstoff über einen längeren Zeitraum gleichmäßig an die Schweißdrüsen abgegeben werden kann. Der AT-Wirkstoff, wie bespielsweise Aluminiumchlorohydrat, hat so länger Zeit, um in dem Schweißwasser komplett gelöst zu werden und somit als Lösung zum Wirkort zu gelangen. Komplett gelöst wird der Wirkstoff optimal freigesetzt. Am Wirkort (in der Schweißdrüse) wird eine deutlich höhere Wirkstoffkonzentration erreicht als bei der Standard-Formulierung mit Cyciomethiconen.

Auch das Pflegegefühl des Anwenders erfindungsgemäßer Zubereitungen verbessert sich gegenüber Cyclomethicon-haltigen Formulierungen aufgrund der erfindungsgemäßen Polyolpartialester.

Neben AT-sprays haben sich die erfindungsgemäßen Polyolpartialester auch in ATstickformulierungen vorteilhaft erwiesen.
In gängigen Marktformel der AT-Sticks sind üblicherweise bis zu 15% PPG-14-Butylether als Maskierungsöl und ca. 40 % Silikonöl (Cyclomethicone) enthalten, die ein relativ starkes Weißeln aufweisen.
Die Silikonölfreien Formulierungen mit den erfindungsgemäßen Polyolpartialestern, insbesondere Partialglyceriden, weißeln demgegenüber deutlich weniger. Auf das als Maskierungsöl eingesetzte PPG-14-Butylether kann bei erfindungsgemäßen Zubereitungen daher ebenfalls verzichtet werden.

Aufgrund des gezeigten Vorteils beim Austausch von Cyclomethiconen durch erfindungsgemäße Polyolpartialester, ist es daher insbesondere von Vorteil den Gehalt an Cyclomethiconen in der Zubereitung kleiner 3 Gew.%, bevorzugt 0 Gew.%, bezogen auf die Gesamtmasse der Zubereitung, zu wählen. Als Cyclomethicone sind insbesondere Cyclopentasiloxane (D5), Cyclohexasiloxane (D6) oder Mischungen daraus zu verstehen.

Bei Aerosolzubereitungen werden häufig Öle zugesetzt, die in der Wirkstofflösung mit dem Treibgas (Propan, Butan, Isobutan) mischbar sind, da ein Öl, das nicht mischbar ist, zu Niederschlägen führt, die im Glasaerosol dazu führen, dass die Wirkstoffpartikel nicht mehr aufzuschütteln sind.
Einige Öle führen zu einem starken "Caking" der Partikel der Wirkstofflösung, so dass sich diese nicht mehr aufschütteln lassen, sondern zu einem festen Kuchen am Boden des Glasaerosols verkleben.

Ein weiterer Vorteil der erfindungsgemäßen Partialester zeigt sich bei wasserfreien AT-Sprays.
Der erfindungsgemäße Vorteil ist, dass die in wasserfreien Zubereitungen vorhandenen Schichtsilikate nicht gesondert aktiviert werden müssen.
Die Aktivierung des Schichtsilikats, z.B. Disteardimonium hectorite, erfolgt durch die erfindungsgemäßen Partialglyceride, beispielsweise Capryl-/ Caprinsäure mit einem Veresterungsgrad von 1.5.
Das Schichtsilikat muss nicht wie bislang üblich durch polare Inhaltsstoffe des Parfümöls aktiviert werden, sondern das, aufgrund der speziellen Hydroxidgruppenverhältnisse, polare Polyolpartialester reagiert schon in einer ähnlichen Konzentration wie das Parfüm direkt mit dem Schichtsilikat und aktiviert dies.

Das Parfüm kann somit erst am Ende der Herstellung der Formulierung zugesetzt werden. Ein vorzeitiger Zusatz des Parfumöls zur Aktivierung der Schichtsilikate kann entfallen. Das hat den Vorteil, dass die Inhaltsstoffe des Parfüms nicht während des Energieeintrags bei der Herstellung, wie durch den Ultraturrax, zerstört werden und nur ein möglichst geringer Anteil des Parfüms verdunstet.
Das Sedimentationsverhalten des Aerosols ist durch die Polyolpartialester ebenfalls positiv beeinflusst, so dass ein Teil der Partikel in der Schwebe bleibt und langsamer sedimentiert. Die Wirkstoffpartikel stehen sofort für die Wirkung zur Verfügung und müssen nicht erst durch den Verbraucher aufgeschüttelt werden.

Durch die spezielle Polarität der Polyolpartialester ist auch eine Einarbeitung sowohl in wasserhaltigen als auch wasserfreien Deo-/AT-Formulierungen möglich.
Die Polyolpartialester weisen aufgrund der erfindungsgemäßen Molverhältnisse der Hydroxygruppen zu den Acylgruppen einen quasi Tensidcharakter auf.
Durch die freien Hydroxidgruppen wird die Polarität der Partialester erhöht, so dass diese Strukturen in den Formulierungen als Emulgator fungieren und somit die Stabilität von klassischen O/W- und W/O-Emulsionen erhöhen.

Dadurch können Wirkstoffe, wie z.B. in einer Mikroemulsion, stabil formuliert werden.

Auch erweisen sich die erfindungsgemäßen Zubereitungen durch eine verbesserte Packmittelkompatibilität aus.
Bekannte Silikonöl-Alternativen sind leicht-flüchtige Kohlenwasserstoffe. Diese haben jedoch einen ähnlichen Löslichkeitsparameter wie das als Packmittel häufig eingesetzte Polypropylen. Sie migrieren in das Polypropylen und führen zu einer Quellung des Packmittels. Nach Lagerung bei +40°C sind diese Packmittel dann teilweise nicht mehr funktionsfähig. Der Deckel lässt sich nicht mehr auf das Stick-Packmittel setzen. Der Verbraucher erkennt einen Spalt zwischen dem inneren und äußeren Packmittel. D.h. ein einfacher Ersatz von Silikonölen ist somit nicht gegeben.

Im Vergleich zu leicht flüchtigen Silikonölen zeigen dagegen eher schwer flüchtige Öle wie C12-15 Alkylbenzoat und Dicaprylylcarbonat ebenfalls eine Packmittelinkompatibilität, besonders nach Lagerung bei erhöhter Temperatur (+40°C).

Die erfindungsgemäßen Zubereitungen zeigen dies Packmittelinkompatibilität, insbesondere bei Packmittel aus PP, nicht.

Weitere Vorteile und Verwendungsmöglichkeiten zeigen die erfindungsgemäßen Zubereitungen in der Weise, dass das bei AT-mitteln bekannte Weißeln verbessert bzw. ganz vermieden wird.

Ebenso führen die erfindungsgemäßen Zubereitungen zu einer verbesserten Dispergierung pulverförmiger Rohstoffe, insbesondere in wasserfreien Suspensionen.

Die kosmetischen und dermatologischen Zubereitungen gemäß der Erfindung können kosmetische Hilfsstoffe und weitere Wirkstoffe enthalten, wie sie üblicherweise in Deodorant- oder Antitranspirantzubereitungen verwendet werden, wie z. B. Konservierungsmittel, Konservierungshelfer, Bakterizide, Parfüme, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate sofern der Zusatz die geforderten Eigenschaften nicht beeinträchtigt oder ausgeschlossen ist.

Bevorzugte Applikationsform für die erfindungsgemäßen Formulierungen mit Polyolpartialestern sind wasserhaltige und wasserfreie Aerosole, die in Aluminiumdosen auf den Markt kommen. Bei den wasserhaltigen Aerosolen sind vor allem die W/O-Emulsionen zu nennen. Bevorzugt ist diese Applikationsform, da sie weltweit die häufigste Form im Bereich Deo/AT darstellt. Aufgrund der sehr guten Packmittelkompatibilität ist auch ein Einsatz in wasserfreien Formulierungen von AT-Sticks zu empfehlen, da es auch nach Lagerung bei +40°C über einen Zeitraum von 6 Monaten kaum zu Packmittelveränderungen kommt. Weiterhin ist ein Einsatz in O/W-Emulsionen in Roll-ons und Pump-Sprays möglich und vorteilhaft.

Die nachfolgenden Beispiele illustrieren erfindungsgemäße Zubereitungen. Die Angaben sind Gewichtsanteile, bezogen auf die Gesamtmasse der jeweiligen Zubereitungen.

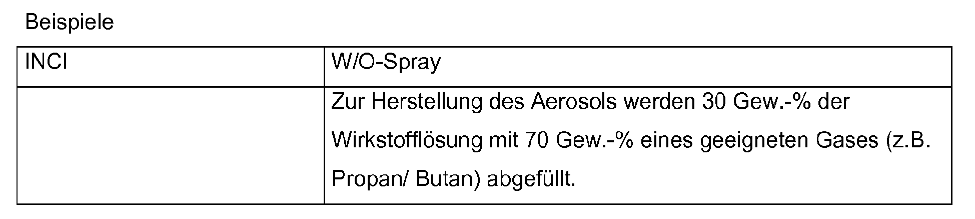

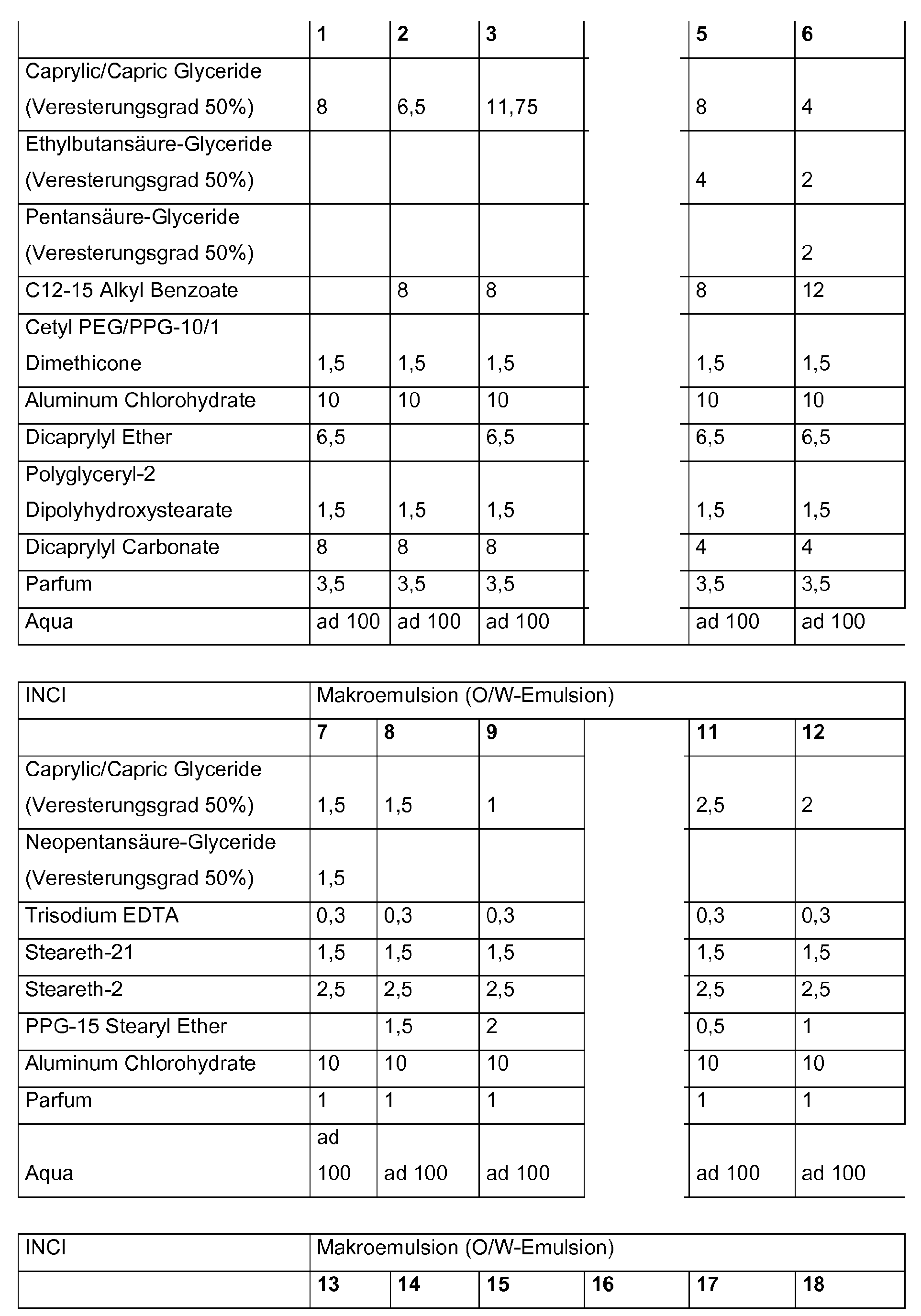

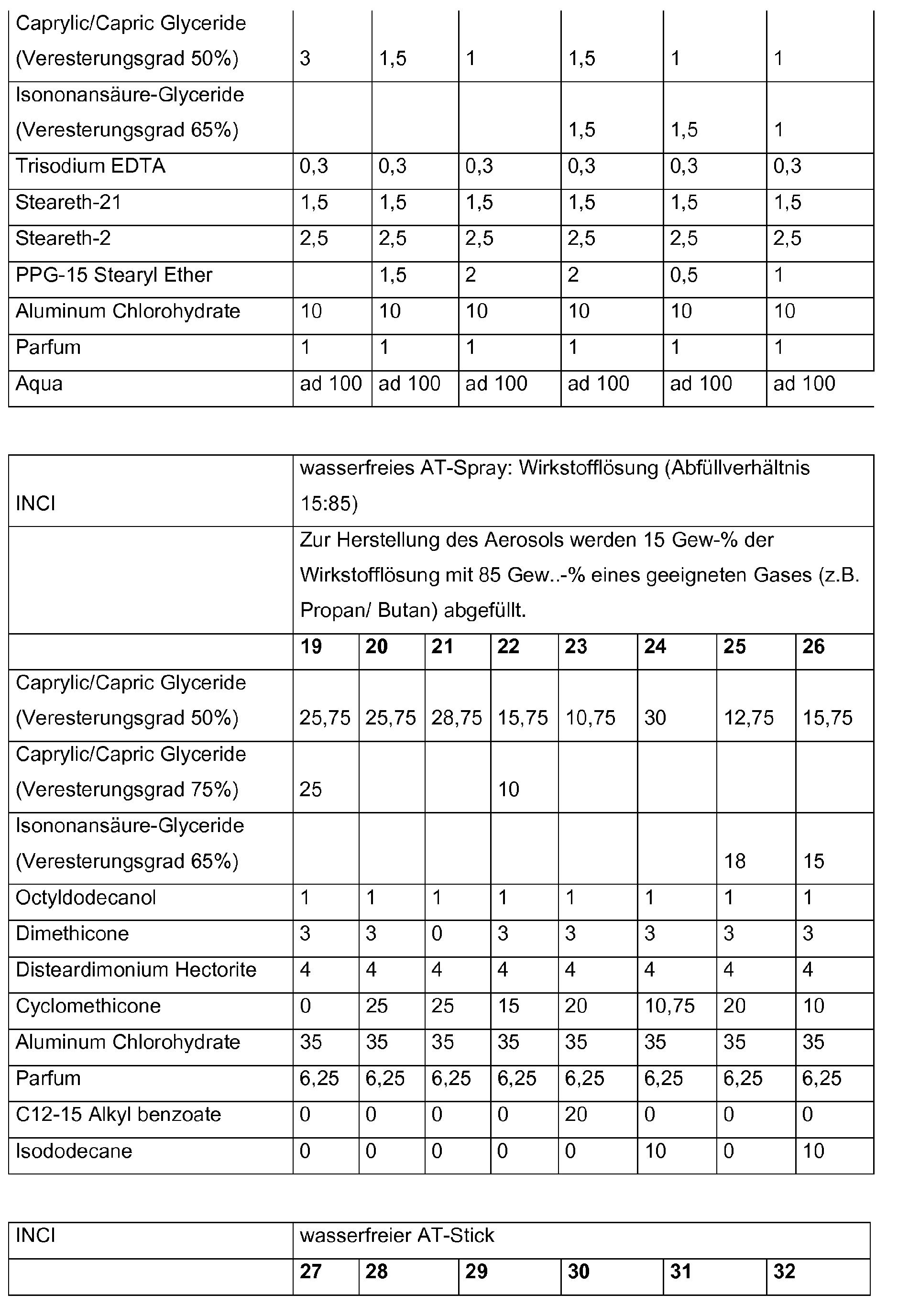

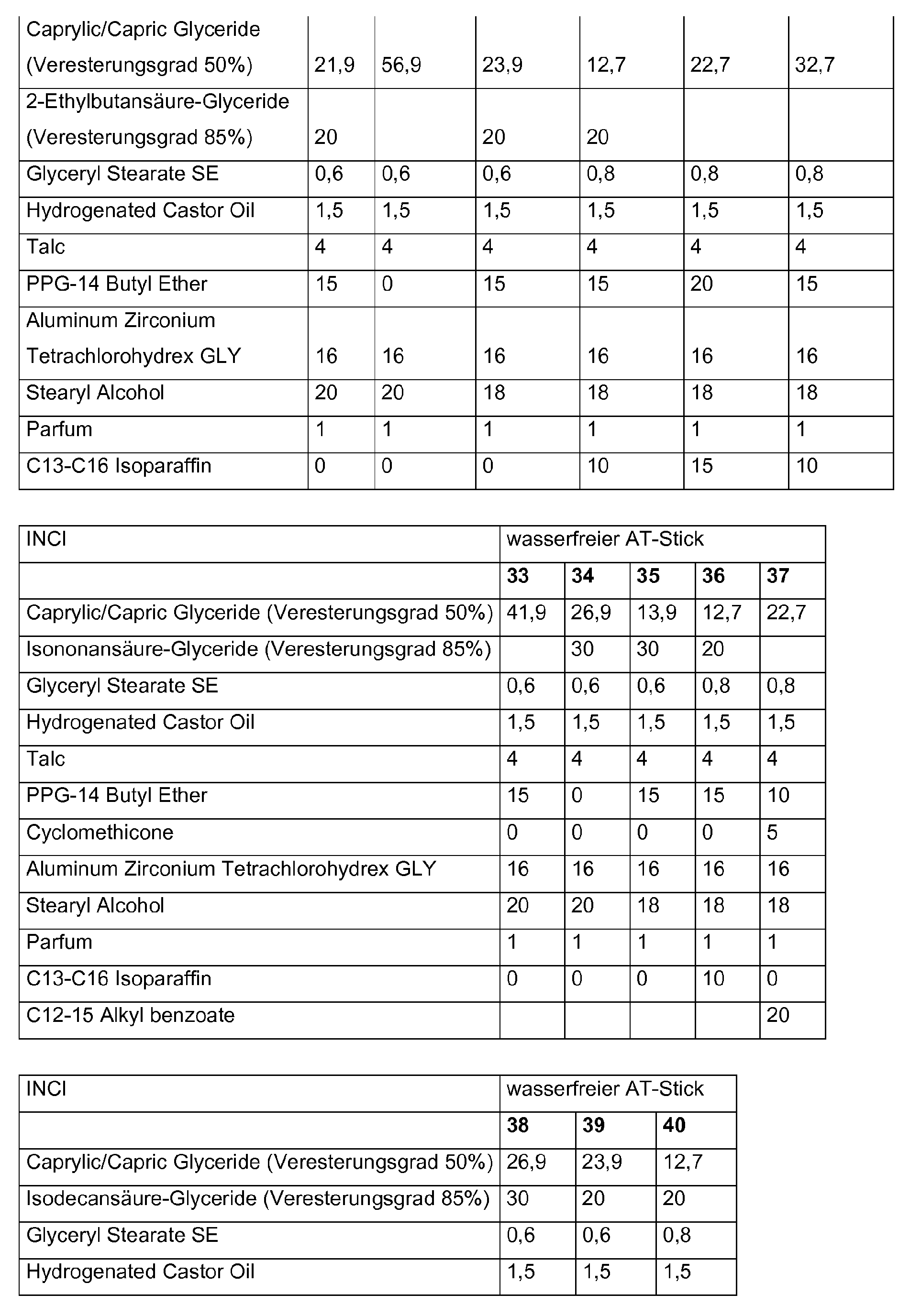

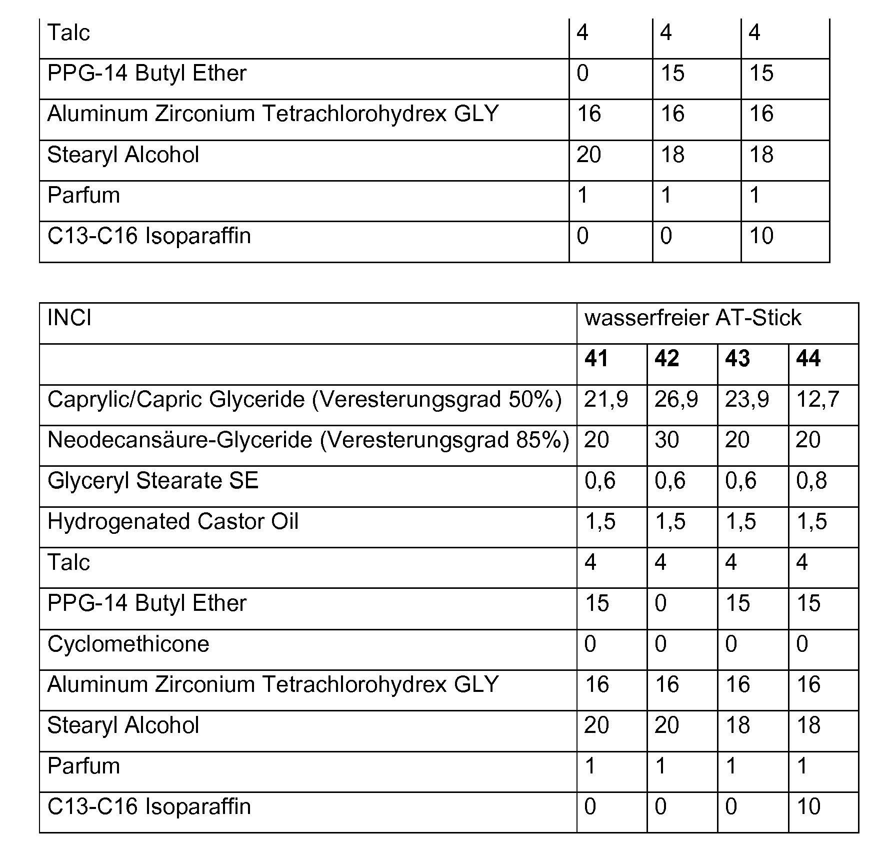

## Patentansprüche

1. Kosmetische oder dermatologische Deodorant- oder Antitranspirantzubereitung umfassend ein oder mehrere Polyolpartialester gebildet aus einem Polyol mit 3 bis 6 Kohlenstoffatomen enthaltend 2 bis 6 OH-Gruppen und mindestens zwei verschiedenen Carbonsäuren enthaltend 5 bis 10 Kohlenstoffatome **dadurch gekennzeichnet, dass** der Veresterungsgrad der Polyolpartialester im Bereich von 35 Mol% bis 90 Mol% liegt, bezogen auf die Gesamtmolzahl an veresterbaren OH-Gruppen.

2. Zubereitung nach Anspruch 1, **dadurch gekennzeichnet, dass** als Polyolpartialester Mischungen mindestens zweier Polyolpartialester, die sich durch ihren Veresterungsgrad unterscheiden, gewählt werden.

3. Zubereitung nach einem der vorstehenden Ansprüche, **dadurch gekennzeichnet, dass** die Carbonsäure 6 bis 10, besonders bevorzugt 8 bis 10, Kohlenstoffatome enthält.

4. Zubereitung nach einem der vorstehenden Ansprüche, **dadurch gekennzeichnet, dass** der Veresterungsgrad der Polyolpartialester im Bereich von 40 bis 83 Mol.%, insbesondere im Bereich von 45 bis 70 Mol.%, liegt

5. Zubereitung nach einem der vorstehenden Ansprüche, **dadurch gekennzeichnet, dass** der Anteil an Polyolpartialester in der Zubereitung im Bereich von 3 bis zu 90 Gew.%, bezogen auf die Gesamtmasse der Zubereitung, gewählt wird.

6. Zubereitung nach Anspruch 5, **dadurch gekennzeichnet, dass** der Anteil an Polyolpartialester in der Zubereitung bis zu 60 Gew.%, bezogen auf die Gesamtmasse der Zubereitung, gewählt wird.

7. Zubereitung nach einem der vorstehenden Ansprüche, **dadurch gekennzeichnet, dass** jeder einzelne Polyolpartialester eines bestimmten Veresterungsgrades nicht mehr als 80 Gew.-% des gesamten Polyolpartialester ausmacht.

8. Zubereitung nach einem der vorstehenden Ansprüche, **dadurch gekennzeichnet, dass** die Polyolpartialester gewählt werden aus den Estern, die oberhalb 22°C und 1 bar flüssig sind.

9. Zubereitung nach einem der vorstehenden Ansprüche, **dadurch gekennzeichnet, dass** der Anteil an Silikonölen, insbesondere Cyclomethiconen, insbesondere Decacyclopentasiloxan in der Zubereitung weniger als 3 Gew.%, insbesondere 0 Gew.%, bezogen auf die Gesamtmasse der Zubereitung, beträgt.

10. Zubereitung nach einem der vorstehenden Ansprüche, **dadurch gekennzeichnet, dass** die Zubereitung eine Spray- oder Stickformulierung ist.

11. Zubereitung nach einem der vorstehenden Ansprüche, **dadurch gekennzeichnet, dass** die Zubereitung eine W/O-Emulsion ist.

12. Zubereitung nach einem der vorstehenden Ansprüche, **dadurch gekennzeichnet, dass** die Zubereitung eine Suspension, bevorzugt wasserfrei, ist.

13. Zubereitung nach einem der vorstehenden Ansprüche umfassend bis zu 30 Gew.% eines oder mehrerer Antitranspirantwirkstoffe, bezogen auf die Gesamtmasse der Zubereitung.

14. Verwendung von ein oder mehreren Polyolpartialester gebildet aus einem Polyol mit 3 bis 6 Kohlenstoffatomen enthaltend 2 bis 6 OH-Gruppen und mindestens zwei verschiedenen Carbonsäuren enthaltend 5 bis 10 Kohlenstoffatome, **dadurch gekennzeichnet, dass** der Veresterungsgrad der Polyolpartialester im Bereich von 35 Mol% bis 90 Mol% liegt, bezogen auf die Gesamtmolzahl an veresterbaren OH-Gruppen, zur Steigerung der Antitranspirantwirksamkeit von kosmetischen Antitranspirantzubereitungen.

## Claims

1. Cosmetic or dermatological deodorant or anti-transpirant preparation comprising one or more polyol partial esters formed from one Polyol having 3 to 6 carbon atoms comprising 2 to 6 OH groups and at least two different carboxylic acids comprising 5 to 10 carbon atoms, **characterized in that** the degree of esterificatian of the polyol partial esters is in the range of 35 mol% to 90 mol%, based on the total number of moles of OH groups that can be esterified.

2. Preparation according to Claim 1, **characterized in that** the polyol partial esters selected are mixtures of at least two polyol partial esters which are differentiated by their degree of esterification.

3. Preparation according to either of the preceding claims, **characterized in that** the carboxylic acid comprises 6 to 10, particularly preferably 8 to 10 carbon atoms.

4. Preparation according to any of the preceding claims, **characterized in that** the degree of esterification of the polyol partial esters is in the range of 40 to 83 mol%, in particular in the range of 45 to 70 mol%.

5. Preparation according to any of the preceding claims, **characterized in that** the proportion of polyol partial esters in the preparation is selected in the range of 3 up to 90% by weight, based on the total mass of the preparation.

6. Preparation according to Claim 5, **characterized in that** the proportion of polyol partial esters in the preparation is selected up to 60% by weight, based on the total mass of the preparation.

7. Preparation according to any of the preceding claims, **characterized in that** each individual polyol partial ester of a particular degree of esterification does not account for more than 80% by weight of the total polyol partial ester.

8. Preparation according to any of the preceding claims, **characterized in that** the polyol partial esters are selected from esters that are liquid above 22°C and 1 bar.

9. Preparation according to any of the preceding claims, **characterized in that** the proportion of silicone oils, particularly cyclomethicones, in particular decacyclopentasiloxane, in the preparation is less than 3% by weight, in particular 0% by weight, based on the total mass of the preparation.

10. Preparation according to any of the preceding claims, **characterized in that** the preparation is a spray or stick formulation.

11. Preparation according to any of the preceding claims, **characterized in that** the preparation is a W/O emulsion.

12. Preparation according to any of the preceding claims, **characterized in that** the preparation is a suspension, preferably free of water.

13. Preparation according to any of the preceding claims comprising up to 30% by weight of one or more anti-transpirant active ingredients, based on the total mass of the preparation.

14. Use of one or more polyol partial esters formed from a polyol having 3 to 6 carbon atoms comprising 2 to 6 OH groups and at least two different carboxylic acids comprising 5 to 10 carbon atoms, **characterized in that** the degree of esterification of the polyol partial esters is in the range of 35 mol% to 90 mol%, based on the total number of moles of OH groups that can be esterified, for increasing the anti-transpirant effectiveness of cosmetic anti-transpirant preparations.

## Revendications

1. Préparation cosmétique ou dermatologique déodorante ou anti-transpirante comprenant un ou plusieurs esters partiels de polyol formés à partir d'un polyol de 3 à 6 atomes de carbone contenant 2 à 6 groupes OH et d'au moins deux acides carboxyliques différents contenant 5 à 10 atomes de carbone, **caractérisée en ce que** le degré d'estérification des esters partiels de polyol se situe dans la plage allant de 35 % en moles à 90 % en moles, par rapport au nombre de moles total de groupes OH estérifiables.

2. Préparation selon la revendication 1, **caractérisée en ce que** des mélanges d'au moins deux esters partiels de polyol, qui diffèrent par leur degré d'estérification, sont choisis en tant qu'esters partiels de polyol,

3. Préparation selon l'une quelconque des revendications précédentes, **caractérisée en ce que** l'acide carboxylique contient 6 à 10, de manière particulièrement préférée 8 à 10, atomes de carbone.

4. Préparation selon l'une quelconque des revendications précédentes, **caractérisée en ce que** le degré d'estérification des esters partiels de polyol se situe dans la plage allant de 40 à 83 % en moles, notamment dans la plage allant de 45 à 70 % en moles.

5. Préparation selon l'une quelconque des revendications précédentes, **caractérisée en ce que** la proportion d'esters partiels de polyol dans la préparation est choisie dans la plage allant de 3 à 90 % en poids, par rapport à la masse totale de la préparation.

6. Préparation selon la revendication 5, **caractérisée en ce que** la proportion d'esters partiels de polyol dans la préparation est choisie jusqu'à 60 % en poids, par rapport à la masse totale de la préparation.

7. Préparation selon l'une quelconque des revendications précédentes, **caractérisée en ce que** chaque ester partiel de polyol individuel d'un degré d'estérification déterminé ne représente pas plus de 80 % en poids de l'ester partiel de polyol total.

8. Préparation selon l'une quelconque des revendications précédentes, **caractérisée en ce que** les esters partiels de polyol sont choisis parmi les esters qui sont liquides au-dessus de 22 °C et 1 bar.

9. Préparation selon l'une quelconque des revendications précédentes, **caractérisée en ce que** la proportion d'huiles de silicone, notamment de cyclométhicones, notamment de décacyclopentasiloxane, dans la préparation est inférieure à 3 % en poids, notamment 0 % en poids, par rapport à la masse totale de la préparation.

10. Préparation selon l'une quelconque des revendications précédentes, **caractérisée en ce que** la préparation est une formulation de pulvérisation ou de bâton.

11. Préparation selon l'une quelconque des revendications précédentes, **caractérisée en ce que** la préparation est une émulsion E/H.

12. Préparation selon l'une quelconque des revendications précédentes, **caractérisée en ce que** la préparation est une suspension, de préférence anhydre.

13. Préparation selon l'une quelconque des revendications précédentes, comprenant jusqu'à 30 % en poids d'un ou de plusieurs agents actifs anti-transpirants, par rapport à la masse totale de la préparation.

14. Utilisation d'un ou de plusieurs esters partiels de polyol, formés à partir d'un polyol de 3 à 6 atomes de carbone contenant 2 à 6 groupes OH et d'au moins deux acides carboxyliques différents contenant 5 à 10 atomes de carbone, **caractérisée en ce que** le degré d'estérification des esters partiels de polyol se situe dans la plage allant de 35 % en moles à 90 % en moles, par rapport au nombre de moles total de groupes OH estérifiables, pour augmenter l'efficacité anti-transpirante de préparations cosmétiques anti-transpirantes.
